# EUROPEAN PATENT APPLICATION

(11) **EP 2 333 545 A2**
(43) Date of publication of application: **15.06.2011**
(21) Application number: 09808425.4
(22) Date of filing: 21.08.2009
(51) Int. Cl.: G01N 33/50

(54) **METHOD FOR INCREASING SENSITIVITY USING LINKER AND SPACER IN CARBON NANOTUBE-BASED BIOSENSOR**

(30) Priority: 22.08.2008 KR 20080082507
(71) Applicant: Sungkyunkwan University Foundation for Corporate Collaboration, Suwon City, Gyeong-gi do 440-746 (KR); M.I. Tech Co., Ltd., Gyeonggi-do 451-864 (KR)
(72) Inventor: SIM, Sang Jun, Seoul 135-506 (KR); KIM, Jun Pyo, Suwon-si Gyeonggi-do 440-828 (KR)
(74) Representative: von Kreisler, Alek
(86) International application number: PCT/KR2009/004685
(87) International publication number: WO 2010/021521

(57) **Abstract**

Disclosed is a method of detecting even a very small amount of a target substance by mixing a linker and a spacer at a suitable ratio and immobilizing the mixture on the surface of carbon nanotubes in a carbon nanotube-based biosensor. This method detects a specific substance at the level of femtomoles and lowers the detection limit of conventional carbon nanotube transistor sensors. Accordingly, the method detects even a very small amount of a target substance, and thus the carbon nanotube-based biosensor is a highly useful sensor which can be used either as a medical sensor for diagnosing diseases or as an environmental sensor.

## Description

### [Technical Field]

The present invention relates to a method of increasing the sensitivity of a carbon nanotube-based biosensor using a linker and a spacer.

### [Background Art]

Carbon nanotubes are a new class of material in which hexagons consisting of six carbon atoms are connected to each other to form a tubular shape. These carbon nanotubes show various unique quantum phenomena due to a quasi-one dimensional quantum structure and have an electrical conductivity similar to that of copper, a thermal conductivity about three times higher than that of diamond (having the highest thermal conductivity among natural materials), a mechanical strength about 100 times higher than that of steel, and a density as low as that of plastics. Due to such properties, carbon nanotubes are widely used in the material field. Also, carbon nanotubes have excellent chemical stability, show semiconducting or conducting properties according to their structure, have a diameter as small as the nanometer scale (10⁻⁹ m) and are elongated and hollow. Due to such properties, carbon nanotubes exhibit excellent device properties in flat display devices, transistors, energy storage materials, etc., and are highly applicable to various electronic devices of nanometer size.

When such nano-sized carbon nanotubes are used as sensors, they can be very sensitive to the external environment to make high-sensitivity measurement possible, can shorten the measurement time, can reduce energy consumption required for operation, can perform a reaction in aqueous solution without modification of protein, and can integrate and miniaturize various kinds of devices due to their nanometer size.

Also, such sensors manufactured using carbon nanotubes perform detection in an electrical manner, and thus have advantages in that they do not require expensive large-scale systems, such as optical analysis systems or other analysis systems, have high sensitivity to eliminate other labeling requirements, can perform real-time analysis and facilitate the development of small-sized and portable sensors.

In the case of general transistors, the electric current of the channel between the source and drain electrodes is controlled by a third gate electrode, whereas, in the case of carbon nanotube transistors, the flow of an electric current is controlled either by a chemical substance to be sensed or by charged molecules, Namely, when gaseous molecules or biomolecules are adsorbed onto the surface of carbon nanotubes, the drainage or accumulation of electric charges will occur, leading to a change in the electrical conductivity of the carbon nanotube devices. Because carbon nanotubes generally behave as p-type semiconductors, the positive gate electrode (that is, the electrical conductivity) decreases due to the adsorption of a positively charged protein.

However, in order to detect biomolecules using a carbon nanotube transistor, the detection reaction should occur within the Debye length. The Debye length is a very important parameter in semiconductor technology and is a measure of the distance over which the charge imbalance is neutralized. For example, if a positively charged sphere is injected into an n-type semiconductor, mobile carriers will be concentrated around the sphere. At a distance of several Debye lengths from the sphere, the positively charged sphere and the electron cloud will appear neutral. When positive (+) charges are inserted into the electron gas or sea, electrons will be concentrated around the inserted charged particles so that the density of electrons will increase. As a result, the charges of the charged particles will be shielded so that they will not influence locations at a distance greater than the Debye length. This phenomenon is known as Debye shielding. The distance to a bundle of electrons concentrated in order to neutralize the charged particles is known as the Debye length.

In order to detect a target substance in a carbon nanotube transistor sensor, a receptor which is immobilized on carbon nanotubes should have small size. Generally, antibodies that are used for the detection of target substances have a size of 10-15 nm, which is much greater than a Debye length of about 3 nm at an ion concentration of 10 mM. Accordingly, these antibodies cannot easily detect a reaction with a target substance, indicating that these antibodies have low sensitivity. In severe cases, the antibodies cannot detect the reaction. Thus, the size of a receptor for a target substance should be very small.

For this reason, as receptors which are immobilized on a carbon nanotube transistor sensor for the detection of a target substance, materials of small size such as aptamers are currently being used. However, because many aptamers for diagnosis of many diseases have not been developed, alternative materials to be used as receptors are required, and the development of new receptors is urgently required. Recently, a method of decreasing sensor sensitivity using antibody fragments as receptors in carbon nanotube transistor sensors was developed. However, this method has an inconvenience in that processes of digesting antibodies using enzymes during surface modification of the carbon nanotube transistor sensor should be carried out.

Accordingly, in order to minimize the number of such processes, the present inventors introduced a linker and a spacer so as to ensure a space between receptors and have developed a method of immobilizing receptors, which bind or react with a target substance, on the linker, thereby completing the present invention.

### [Disclosure]

### [Technical Problem]

It is an object of the present invention to provide a carbon nanotube-based biosensor capable of detecting even a very small amount of a target substance, a method of detecting the target substance using the same, and a fabrication method thereof.

### [Technical Solution]

To achieve the above object, the present invention provides a carbon nanotube-based biosensor comprising a linker and a spacer, a method of detecting a target substance using the same, and a fabrication method thereof.

In one aspect, the present invention provides a carbon nanotube-based biosensor comprising: a spacer and a linker, which are immobilized on the surface of carbon nanotubes of a carbon nanotube transistor; and a bioreceptor immobilized on the linker, wherein one end of the linker is a pyrene group or graphite, and the spacer is a compound having a structure represented by the following formula 1:

[Formula 1] X-L-Y

wherein X is the pyrene group or graphite; L is (CH₂)n wherein n is an integer ranging from 1 to 4; and Y is a hydroxyl group (-OH).

The pyrene group or graphite that is one end of the spacer may be adsorbed on carbon nanotubes, and the hydroxyl group (-OH) that is the other end of the spacer can prevent nonspecific adsorption.

In one embodiment, X in the spacer of formula 1 may be the pyrene group. More specifically, the spacer may be 1-pyrenebutanol.

The pyrene group or graphite that is one end of the linker may be adsorbed onto carbon nanotubes.

In one embodiment, the linker may be 1-pyrenebutanoic acid succinimidyl ester.

The mixing ratio of the linker and the spacer is preferably 1:1 to 1:9, and more preferably 1:3.

The bioreceptor may be, but is not limited to, an antibody, an enzyme, a protein, a peptide, an amino acid, an aptamer, a lipid, a cofactor or a carbohydrate. Preferably, it may be a monoclonal antibody, a polyclonal antibody or an antibody-binding site fragment. The target substance binding to the bioreceptor serves as a gate.

In one embodiment, the present invention provides a carbon nanotube-based transistor biosensor wherein the linker is 1-pyrenebutanoic acid succinimidyl ester; the spacer is 1-pyrenebutanol; and the bioreceptor is anti-human IgG F(ab')₂.

The channel region of the carbon nanotube transistor preferably has a structure in which single-wall or multi-wall carbon nanotubes are entangled with each other.

The single-wall carbon nanotubes are preferably carbon nanotubes having a diameter of 2-4 nm, and the multi-wall carbon nanotubes are preferably carbon nanotubes having a diameter of 50 nm or less, but the scope of the present invention is not limited thereto.

In another aspect, the present invention provides a method of detecting a target substance using a carbon nanotube-based biosensor, the method comprising the steps of:
(i) immobilizing a linker and a spacer on carbon nanotubes in the channel region of a carbon nanotube transistor;
(ii) immobilizing a bioreceptor which is able to bind the target substance on the linker;
(iii)measuring a change in the electrical conductivity of the carbon nanotube transistor; and
(iv) detecting or quantifying the target substance based on the data of the change in the electrical conductivity;
wherein one end of the linker is a pyrene group or graphite, and the spacer is a compound having a structure represented by the following formula 1:

[Formula 1] X-L-Y

wherein X is the pyrene group or graphite; L is (CH₂)n wherein n is an integer ranging from 1 to 4; and Y is a hydroxyl group (-OH).

In yet another aspect, the present invention provides a method for fabricating a carbon nanotube-based biosensor, the method comprising the steps of:
(i) immobilizing a linker and a spacer on carbon nanotubes in the channel region of a carbon nanotube transistor; and
(ii) immobilizing a bioreceptor which is able to bind the target substance on the linker;
wherein one end of the linker is a pyrene group or graphite, and the spacer is a compound having a structure represented by the following formula 1:

[Formula 1] X-L-Y

wherein X is the pyrene group or graphite; L is (CH₂) n wherein n is an integer ranging from 1 to 4; and Y is a hydroxyl group (-OH).

### [Advantageous Effects]

According to the present invention, the spacer is used to create a space between receptors so as to reduce steric hindrance between proteins. Thus, the accesibility of a target substance near the carbon nanotubes can be improved, thereby increasing the sensitivity of the sensor. The greatest advantage of the biosensor comprising the linker and the spacer on the surface of carbon nanotubes is that sensitivity (minimum detection limit value) required for the sensor can be obtained even when relatively large receptors are used. Accordingly, the sensitivity of the sensor can be increased so as to detect a low concentration of a target substance, even when the size of receptors is large.

### [Description of Drawings]

FIG. 1 is a conceptual view of a carbon nanotube transistor biosensor for detecting a very small amount of a target substance, in which a linker and a spacer are immobilized on the surface of carbon nanotubes.
FIG. 2 is a graphic diagram showing current characteristics obtained when the target substance human IgG was detected on surface A.
FIG. 3 is a graphic diagram showing current characteristics obtained when the target substance human IgG was detected on surface B.
FIG. 4 is a graphic diagram showing current characteristics obtained when the target substance human IgG was detected on surface C.
FIG. 5 is a graphic diagram showing current characteristics obtained when the target substance human IgG was detected on surface D.
FIG. 6 is a graphic diagram showing current characteristics obtained when the non-target substance BSA (bovine serum antigen) was detected on surface C for nonspecific adsorption.

### [Best Mode]

In a preferred embodiment, carbon nanotubes provided in carbon nanotube transistor channels consist of single-wall carbon nanotubes which are entangled like cobwebs. Also, a spacer and a linker are immobilized on the surface of carbon nanotubes provided between the channels to control the distance between receptors, so that a target substance can easily approach the surface of the carbon nanotubes. The mixing ratio between the spacer and the linker is preferably 1:1, 1:3, or 1:9.

Hereinafter, the present invention will be described in further detail with reference to the accompanying drawings.

The present invention relates to a biosensor obtained by adsorbing a linker and a spacer on the surface of carbon nanotubes of a carbon nanotube transistor at a suitable ratio to modify the surface of the carbon nanotubes. The biosensor of the present invention can detect either antigen-antibody reactions, which are used for the diagnosis of general diseases, or even a very low concentration of pathogens in the environmental field.

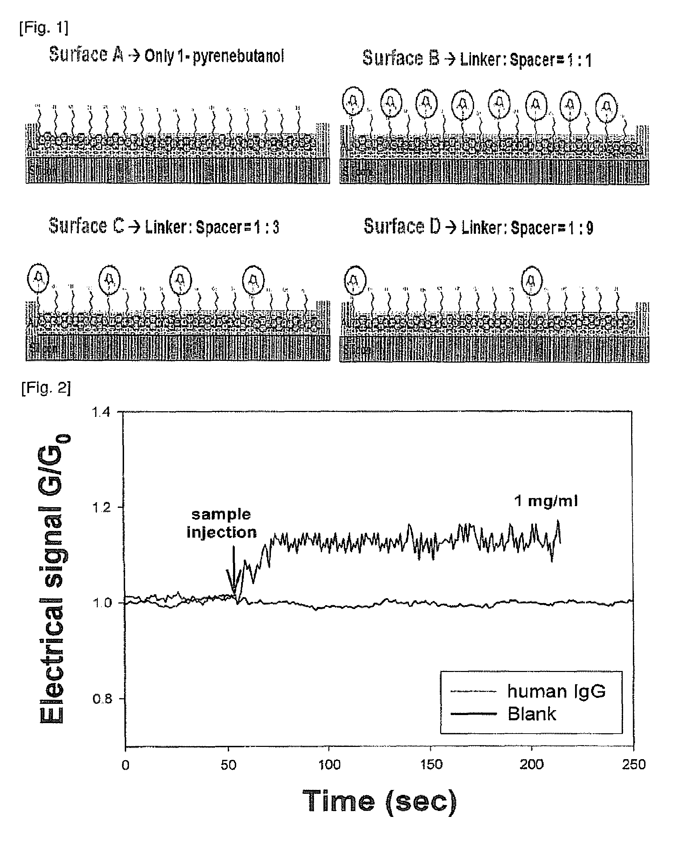

FIG. 1 is a conceptual view showing a carbon nanotube transistor biosensor for detecting a very small amount of a target substance, in which a linker and a spacer are immobilized on the surface of carbon nanotubes at a suitable ratio.

Generally, the principle of carbon nanotube transistor sensors is that a substance binding to a target substance is immobilized on the surface of carbon nanotubes and that the target substance is dropped on the surface to observe a change in an electrical signal. In FIG. 1, surface A is a surface obtained by immobilizing only the spacer 1-pyrenebutanol on carbon nanotubes without a linker, surface B is a surface obtained by mixing a linker and a spacer at a ratio of 1:1 and immobilizing the mixture on carbon nanotubes, surface C is a surface obtained by mixing a linker and a spacer at a ratio of 1:3 and immobilizing the mixture on carbon nanotubes, and surface D is a surface obtained by mixing a linker and a spacer at a ratio of 1:9 and immobilizing the mixture on carbon nanotubes.

FIG. 2 is a graphic diagram showing a change in an electrical signal, obtained by immobilizing only a spacer on the surface of carbon nanotubes without using a linker and then allowing the target protein IgG to react with the surface of the carbon nanotubes. In view of the characteristics of CNT-FET (Carbon Nano Tube-Field Effect Transistor), when a target protein is bound to the CNT-FET, the electrical signal of the CNT-FET should be reduced. However, as shown in FIG. 2, the electrical signal of the CNT-FET on which only the spacer was immobilized was increased. This suggests that the spacer is very effective in preventing nonspecific adsorption by interfering with the approach of the protein to the surface of the CNT-FET.

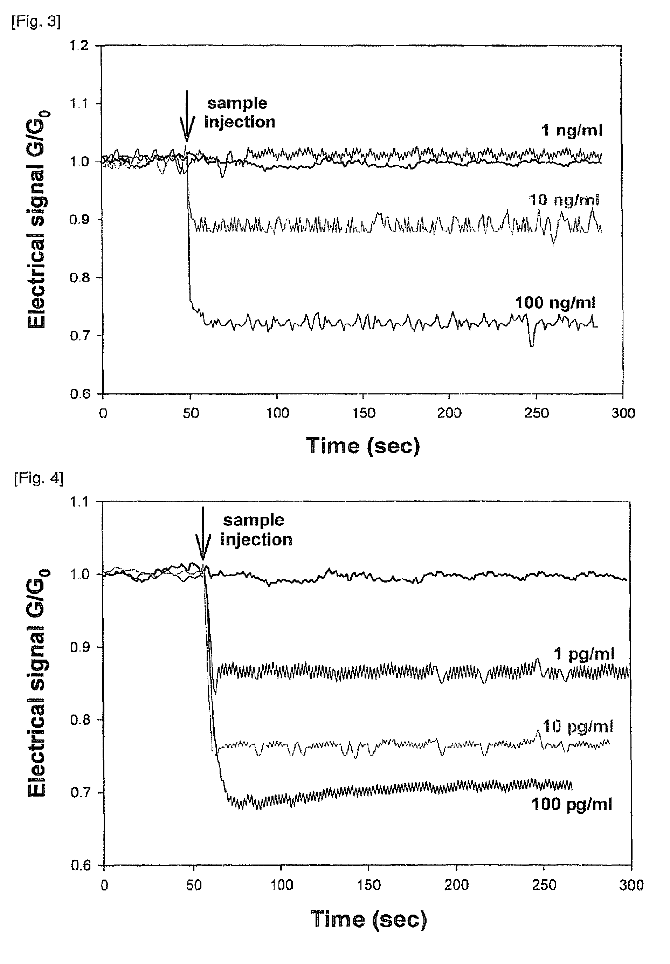

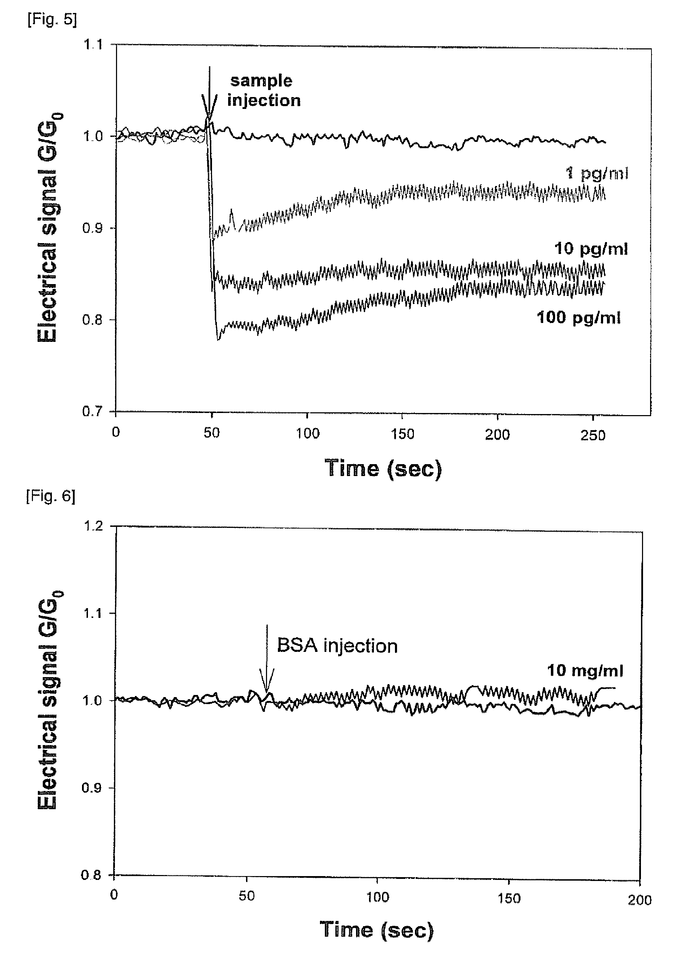

FIGS. 3 to 5 show electrical signals which changed when a target protein was allowed to react with surfaces B, C and D, respectively. As can be seen therein, the sensitivity of the CNT-FET sensor changed depending on the ratio of the linker to the spacer. When the ratio of the linker to the spacer was 1:1, the minimum detection limit was 10 ng/ml, and when the ratio was 1:3, the minimum detection limit was reduced to 1 pg/ml. However, when the ratio was 1:9, the minimum detection limit was 1 pg/ml, like the case in which the ratio was 1:3, but the detection range became narrower than the case in which the ratio was 1:3. This is believed to be because the number of receptors decreases compared to the case in which the ratio was 1:3.

The method according to the present invention is a method capable of detecting a specific substance at the level of femtomoles and can lower the detection limit of conventional carbon nanotube transistor sensors. Accordingly, the method of the present invention can detect a very small amount of a target substance, and thus will be used for the diagnosis of diseases.

Hereinafter, preferred examples are provided for a better understanding of the present invention. It is to be understood, however, that these examples are for illustrative purposes only and are not to be construed to limit the scope of the present invention.

### Example 1: Detection of a target substance on a surface having a linker-to-spacer ratio of 1:1

1 mM of the linker 1-pyrenebutanoic acid succinimidyl ester and 1 mM of the spacer 1-pyrenebutanol were mixed with each other at each of ratios of 1:1, 1:3 and 1:9, and the mixture was immobilized on the surface of networked carbon nanotubes constituting the channel region of a carbon nanotube transistor, thereby forming a single molecular layer on the surface of the carbon nanotubes. Then, the channel region of the carbon nanotube transistor sensor was washed with methanol and immobilized with 20 µg/ml of F(ab')₂-type anti-human IgG (Sigma, USA). Then, the carbon nanotube transistor sensor was washed several times with PBS buffer. After the carbon nanotube transistor sensor had been dried with nitrogen gas, it was allowed to react with 1-1000 ng/ml of IgG (Sigma, USA), thereby measuring the current characteristics of the carbon nanotube transistor sensor.

As can be seen in FIG. 3, when 1 ng/ml of IgG was allowed to react, the electrical signal slightly increased compared to a control, and when 10 ng/ml and 100 ng/ml of IgG were allowed to react, the electrical signal rapidly decreased compared to the control. This detection sensitivity had the minimum detection concentration identical to the value when only the linker was used without using the spacer. Thus, the detection limit of the CNT-FET sensor having a linker-to-spacer ratio of 1:1 was 10 ng/ml. However, a sensor having this detection limit is impossible to use as a disease diagnostic sensor for detecting a few ng/ml of a target substance.

### Example 2 : Detection of a target substance on a surface having a linker-to-spacer ratio of 1:3

According to the method of Example 1, a single molecular layer was formed on the surface of carbon nanotubes in such a manner that the ratio of the linker to the spacer was 1:3 so as to increase the distance between receptors compared to Example 1. As shown in FIG. 4, the target substance human IgG was allowed to react with the CNT-FET sensor, on which the linker and the spacer have been immobilized at a ratio of 1:3, at a concentration ranging from 100 fg/ml to 1000 pg/ml, At this time, a change in the electric current between the source and drain electrodes was measured. As a result, when 100 fg/ml of human IgG was allowed to react, the electric current slightly increased compared to a control, and when 1 pg/ml or more of human IgG was allowed to react, the electric current gradually decreased with an increase in the concentration of human IgG. However, when 1000 pg/ml of human IgG was allowed to react, the electric current did not decrease compared to the case in which 100 pg/ml of human IgG was allowed to react.

The antigen-antibody immune reaction in the method of Example 1 shows a serious limitation in diagnosing diseases using the carbon nanotube transistor sensor. For example, PSA (prostate-specific antigen) protein whose level increases in the case of prostate cancer has a value of 4 ng/ml or less in the case of normal persons, and a PSA protein level of more than 4 ng/ml can be diagnosed as prostate cancer. If the PSA protein detection limit of the method is 10 ng/ml, cancer will not be detected even when the cancer progressed. Because another cancer indicator has a value of 10 ng/ml or less in the case of normal persons, the detection limit of the carbon nanotube transistor biosensor should be further lowered in order to diagnose disease using the biosensor. In the case of Example 2 in which the ratio of the linker to the spacer on the surface of carbon nanotubes was 1:3, when the target substance human IgG was detected on the surface of the carbon nanotubes, the detection limit was significantly lowered to 1 pg/ml compared to the case of Example 1 in which the linker and the spacer were immobilized at a ratio of 1:1. Accordingly, the sensitivity of the sensor can be significantly lowered depending on how the surface of carbon nanotunbes is designed.

### Example 3: Detention of target substance on a surface having a linker-to-spacer ratio of 1:9

According to the method of Example 1, a single molecular layer was formed on the surface of carbon nanotubes in such a manner that the ratio of the linker to the spacer was 1:9 so as to increase the distance between receptors compared to Example 1. Also, human IgG was allowed to react with the carbon nanotube transistor channel at a concentration ranging from 100 fg/ml to 1 ng/ml. At this time, a change in the electric current between the source and drain electrodes was measured. As a result, when 100 fg/ml of IgG was allowed to react, the electric current slightly increased compared to a control, indicating that this concentration of IgG was not detected, and when 1 pg/ml or more of IgG was allowed to react, the electric current gradually decreased with an increase in the concentration of IgG. However, when the concentration of IgG was 100 pg/ml or more, the electric current decreased rather than increased. This is believed to be because the number of receptors immobilized on the surface having a linker-to-spacer ratio of 1:9 decreased by about three times compared to that on the surface having a linker-to-spacer ratio of 1:3. Also, a graph of the electric current rapidly decreased, and then increased again. This is believed to be because the charged protein approached the spacer portion of the carbon nanotubes, and then washed out without binding to the receptors. Accordingly, the detection range of the surface having a linker-to-spacer ratio of 1:3 was 1-100 pg/ml, whereas the the detection range of the surface having a linker-to-spacer ratio of 1:9 became significantly narrower to 1-10 pg/ml.

### [Industrial Applicability]

As described above, according to the present invention, the detection limit of the CNT-FET biosensor can be lowered to a level of 1 pg/ml by modifying the surface of carbon nanotubes of the biosensor using the linker and the spacer. The inventive carbon nanotube-based biosensor comprising the linker and the sensor can detect even a very small amount of a target substance, and thus is a highly useful sensor capable of substituting for either conventional medical sensors for diagnosing diseases, or environmental sensors.

## Claims

1. A carbon nanotube-based biosensor comprising:
a spacer and a linker, which are immobilized on the surface of carbon nanotubes of a carbon nanotube transistor; and
a bioreceptor immobilized on the linker;
wherein one end of the linker is a pyrene group or graphite, and the spacer is a compound having a structure represented by the following formula 1:
[Formula 1] X-L-Y
wherein X is the pyrene group or graphite; L is (CH₂)ₙ wherein n is an integer ranging from 1 to 4; and Y is a hydroxyl group (-OH).

2. The carbon nanotube-based biosensor of claim 1, wherein X in the spacer of formula 1 is the pyrene group.

3. The carbon nanotube-based biosensor of claim 1, wherein the linker is 1-pyrenebutanoic acid succinimidyl ester.

4. The carbon nanotube-based biosensor of claim 1, wherein the bioreceptor is an antibody, an enzyme, a protein, a peptide, an amino acid, an aptamer, a lipid, a cofactor or a carbohydrate.

5. The carbon nanotube-based biosensor of claim 1, wherein the channel region of the carbon nanotube transistor has a structure in which single-wall or multi-wall carbon nanotubes are entangled with each other.

6. The carbon nanotube-based biosensor of claim 5, wherein the single-wall carbon nanotubes are carbon nanotubes having a diameter of 2-4 nm, and the multi-wall carbon nanotubes are carbon nanotubes having a diameter of 50 nm or less.

7. The carbon nanotube-based biosensor of claim 1, wherein the spacer is 1-pyrenebutanol.

8. The carbon nanotube-based biosensor of claim 1, wherein the linker is 1-pyrenebutanoic acid succinimidyl ester; the spacer is 1-pyrenebutanol; and the bioreceptor is anti-human IgG F(ab')₂.

9. The carbon nanotube-based biosensor of any one of claims 1 to 8, wherein the mixing ratio between the linker and the spacer is 1:1 to 1:9.

10. The carbon nanotube-based biosensor of any one of claims 1 to 8, wherein the mixing ratio between the linker and the spacer is 1:3.

11. A method of detecting a target substance using a carbon nanotube-based biosensor, the method comprising:
(i) immobilizing a linker and a spacer on carbon nanotubes in the channel region of a carbon nanotube transistor;
(ii) immobilizing a bioreceptor which is able to bind the target substance on the linker;
(iii) measuring a change in the electrical conductivity of the carbon nanotube transistor; and
(iv) detecting or quantifying the target substance based on the data of the change in the electrical conductivity,
wherein one end of the linker is a pyrene group or graphite, and the spacer is a compound having a structure represented by the following formula 1:
[Formula 1] X-L-Y
wherein X is the pyrene group or graphite; L is (CH₂) n wherein n is an integer ranging from 1 to 4; and Y is a hydroxyl group (-OH).

12. The method of claim 11, wherein X in the spacer of formula 1 is the pyrene group.

13. The method of claim 11, wherein the linker is 1-pyrenebutanoic acid succinimidyl ester.

14. The method of claim 11, wherein the bioreceptor is an antibody, an enzyme, a protein, a peptide, an amino acid, an aptamer, a lipid, a cofactor or a carbohydrate.

15. The method of claim 11, wherein the channel region of the carbon nanotube transistor has a structure in which single-wall or multi-wall carbon nanotubes are entangled with each other

16. The method of claim 15, wherein the single-wall carbon nanotubes are carbon nanotubes having a diameter of 2-4 nm, and the multi-wall carbon nanotubes are carbon nanotubes having a diameter of 50 nm or less.

17. The method of claim 11, wherein the spacer is 1-pyrenebutanol.

18. The method of claim 11, wherein the linker is 1-pyrenebutanoic acid succinimidyl ester; the spacer is 1-pyrenebutanol; and the bioreceptor is anti-human IgG F(ab')₂.

19. The method of any one of claims 11 to 18, wherein the mixing ratio between the linker and the spacer is 1:1 to 1:9.

20. The method of any one of claims 11 to 18, wherein the mixing ratio between the linker and the spacer is 1:3.

21. A method for fabricating a carbon nanotube-based biosensor, the method comprising the steps of:
(i) immobilizing a linker and a spacer on carbon nanotubes in the channel region of a carbon nanotube transistor; and
(ii) immobilizing a bioreceptor which is able to bind the target substance on the linker;
wherein one end of the linker is a pyrene group or graphite, and the spacer is a compound having a structure represented by the following formula 1:
[Formula 1] X-L-Y
wherein X is the pyrene group or graphite; L is (CH₂)n wherein n is an integer ranging from 1 to 4; and Y is a hydroxyl group (-OH).

22. The method of claim 21, wherein X in the spacer of formula 1 is the pyrene group.

23. The method of claim 21, wherein the linker is 1-pyrenebutanoic acid succinimidyl ester.

24. The method of claim 21, wherein the bioreceptor is an antibody, an enzyme, a protein, a peptide, an amino acid, an aptamer, a lipid, a cofactor or a carbohydrate.

25. The method of claim 21, wherein the channel region of the carbon nanotube transistor has a structure in which single-wall or multi-wall carbon nanotubes are entangled with each other.

26. The method of claim 25, wherein the single-wall carbon nanotubes are carbon nanotubes having a diameter of 2-4 nm, and the multi-wall carbon nanotubes are carbon nanotubes having a diameter of 50 nm or less.

27. The method of claim 21, wherein the spacer is 1-pyrenebutanol.

28. The method of claim 21, wherein the linker is 1-pyrenebutanoic acid succinimidyl ester; the spacer is 1-pyrenebutanol; and the bioreceptor is anti-human IgG F(ab¹)₂.

29. The method of any one of claims 21 to 28, wherein the mixing ratio between the linker and the spacer is 1:1 to 1:9.

30. The method of any one of claims 21 to 28, wherein the mixing ratio between the linker and the spacer is 1:3.
